(19) Europäisches Patentamt
European Patent Office
Office européen des brevets

(11) Publication number: 0 046 947
A1

# (12) EUROPEAN PATENT APPLICATION

(21) Application number: 81106520.0

(22) Date of filing: 21.08.81

(51) Int. Cl.[3]: C 07 B 29/00
C 08 G 65/28, C 08 G 65/26
B 01 J 23/02, C 07 C 41/03
C 07 C 43/13, C 07 C 45/64
C 07 C 85/24, C 07 C 102/00
C 07 C 148/00, C 07 C 51/367

(30) Priority: 29.08.80 US 182723

(43) Date of publication of application: 10.03.82 Bulletin 82/10

(84) Designated Contracting States: BE DE FR GB SE

(71) Applicant: CONOCO INC.
1000 South Pine Street P.O. Box 1267
Ponca City Oklahoma 74601(US)

(72) Inventor: Kang, Yang
2501 Robin Road
Ponca City Oklahoma 74601(US)

(72) Inventor: Nield, Gerald Lee
837 Edgewood
Ponca City OK 74601(US)

(72) Inventor: Washecheck, Paul Howard
2801 Canterbury
Ponca City OK 74601(US)

(74) Representative: Patentanwälte Dipl.-Ing. A. Grünecker, Dr.-Ing. H. Kinkeldey, Dr.-Ing. W. Stockmair, Dr. rer. nat. K. Schumann, Dipl.-Ing. P.H. Jakob, Dr. rer. nat. G. Bezold Maximilianstrasse 43
D-8000 München 22(DE)

## (54) Strontium and barium containing alkoxylation systems.

(57) Basic compounds and salts of strontium and barium, and the metals themselves, are used to catalyze the formation of propylene oxide, ethylene oxide and propylene oxide/ethylene oxide adducts of a variety of organic compounds. Materials containing polvols, carboxylic acids, amines, phenols, mercaptans, alkanols, and amides can be reacted to form these adducts. Reactions products have peaked distributions, low pour points, and low levels of unreacted starting materials. Reactions are normally carried out at temperatures of from about 90 to about 260°C.

EP 0 046 947 A1

# STRONTIUM AND BARIUM CONTAINING ALKOXYLATION SYSTEMS

This invention relates to the production of alkoxylated organic compounds by reacting said compounds in the presence of barium-containing or strontium-containing catalysts or promoted barium-containing and strontium-containing catalysts. More particularly, this invention relates to the production of alkoxylated organic compounds by reacting said compounds in the presence of these catalysts with adducting materials such as ethylene oxide and propylene oxide.

The general reaction of a variety of organic material together with an adducting material such as ethylene oxide or propylene oxide to form alkoxylated materials is known in the art. For example, U.S. Patent 2,683,087 discloses that water adsorption by paper articles is improved by the use of amine adducts of ethylene oxide. Another pulp and paper use is disclosed in "Etho-Chemicals" by Armour and Company which discloses that ethoxylated resin fatty acid or ethoxylated stearic acid are incorporated in formulations used for deinking wastepaper. Redeposition of the ink particles is reduced by the use of these surfactants and stabilizers. British Patent 847,714 teaches the processing of pre-hydrolyzed sulfate wood pulp into viscose by incorporating a propylene oxide/ethylene oxide adduct of ethylene diamine. French Patent 1,122,729 discloses the use of an acylarylpolyglycol adduct to the viscose pulp or slurry. U.S. Patent 2,392,103 shows the use of alkyl thioether adducts to viscose pulp. British Patent 815,508 discloses the addition to viscose pulp of an adduct containing at least 7 moles of ethylene oxide per mole of an aliphatic acid, amide, or aldehyde. Elöd, et al discloses the advantages obtained from using various surfactants during the preparation of viscose.

Belgium Patent 555,529 discloses an anti-static agent for synthetic fibers which is produced by esterifying one mole of lauric acid with one mole of an ethoxylated glycerol. British Patent 763,215 suggests ethoxylated organic sulfamides as anti-static agents for textiles. British Patent 779,491 discloses anti-static treatment of textiles with an ether, amine, or thioether adduct having a mole

weight of at least 2000 plus a sulfated fatty acid salt or its ester. British Patent 774,035 discloses that dyeing of wool with metal complex dyes is improved in the presence of an anionic surfactant and an alkylene oxide adduct such as 60 etho-stearic acid amide. British Patent 738.379 discloses that textiles are impregnated against soiling with a solution of a stearic acid adduct of about 6000 molecular weight.

British Patent 705,117 discloses an emulsifier combination for pesticides comprising a mixture which includes a tall oil or dodecyl mercaptan adduct. British Patent 858.687 teaches emulsifiable concentrates of pentachlorophenol which includes 1 to 5% of 3 etho-alkyl propylene diamines. U.S. Patent 2.552,187 shows emulsifiable concentrates comprising a biologically active agent, an alkyl mercaptan adduct, and other materials.

Polyol ethoxylates find uses in foods and feeds. As an example. U.S. Patent 2,674.534 discloses the use of sorbitol laurate and sorbitol oleate adducts in the coating of ice cream bars. The use of ethoxylated fatty acids such as 8 etho-coco fatty acid has been recognized as a feed additive to accelerate animal growth.

Alkylene oxide adducts are also used in the leather industry in formulations for tanning, dyeing, and lubricating leathers. U.S. Patent 2,893,811 shows the use of 12 to 18 carbon atom etho-fatty amines for dyeing of vegetable-tanned leathers. U.S. 2,946,649 shows that adducts of higher molecular weight sulfonamides are effective tanning agents.

Adducts of these materials also have a variety of uses in metal working industries. Ester, ether, and amine adducts are the products used most frequently. Cleaning of metal via degreasing using alkali is practiced extensively. Because of the low foaming characteristics, adducts of esters, ethers, and amines are preferred over many other agents when metals are sprayed by alkaline media. Efficiency of a degreasing bath is improved by the addition of a lauric acid adduct.

Ethylene oxide adducts such as sorbitan monostearate adducts have been found useful in pharmaceutical and cosmetic preparations and are used to provide activities such as drug carriers, emulsifiers, and solublizers. Some adducts have their own pharmacological actions.

Oleic acid adducts plus other substances impart mechanical and chemical stability to latex rubber.

U.S. Patent 3,088,796 discloses a formulation which imparts protection to metal surfaces against corrosion by a flowing stream, one component of such formulations being an ethoxylated alkylamine. U.S. Patent 3,060,132 discloses that a Ziegler catalyst can be modified to prevent the loss of activity on storage by the addition of etho-dodecyl mercaptan.

German 1,142,465 discloses that polyglycol esters derived from reacting ethylene oxide and/or propylene oxide with aliphatic or aromatic carboxylic acids prevents carburetor icing when added to gasoline. Other uses for alkylene oxide and propylene oxide adducts are given in Schonfeldt, Surface Active Ethylene Oxide Adducts, Pergammon Press, Oxford England, 1969 pages 386 through 631.

These reactions are normally carried out using alkaline catalysts, which invariably produce a distribution of various adducts. In surfactant applications, an adduct containing too few adducting molecules is not effective because of poor solubility, while one with too many adducting molecules is undesirable because surface tension reduction per unit mass decreases drastically as the molecular weight increases. Therefore, most desirable adducts deal with as sharp a distribution in the desired mole adduct range as possible. Normally, an acid catalyzed reaction will produce such alkoxylates, but such catalysts also produce higher levels of harmful side products which must be separated and removed prior to use.

Therefore, great benefit would be provided by a catalyst system which provides low by-product levels while retaining the desired mole-adduct distribution of acid catalysts. Such catalysts could promote the narrowing of

the product distribution curve which could contribute significantly to the intrinsic value of the adducts produced.

It is therefore an object of the present invention to provide a catalyst and method for obtaining alkoxylated adducts of a variety of organic materials using strontium-containing and/or barium-containing catalysts. Other objects will become apparent to those skilled in this art as the description proceeds.

It has now been discovered according to the present invention that materials selected from the group consisting of polyols, aldehydes, ketones, amides, amines, acids, and mercaptans can be alkoxylated by contacting these materia with an alkoxylating agent in the presence of a catalyst selected from the group consisting of strontium metal, barium metal, strontium oxide, barium oxide, strontium hydroxide, hydrated strontium hydroxide, barium hydroxide, hydrated barium hydroxide, strontium hydride, barium hydride, or mixtures of these wherein the alkoxylation is carried out at temperatures of from about 90°C to about 260°C.

The process of the instant invention can be carried out at ambient pressure. However, pressures up to about 100 pounds per square inch gauge, (psig) can also be used. Pressures below about 60 psig are preferred. Pressures below ambient can also be used but are not preferred. It is clear that while pressure or lack of pressure is not a detriment to the process of the present invention, it is simply more convenient to carry out the reaction in the pressure range of from about atmospheric to about 100 psig.

The instant invention is normally carried out at temperatures of from about 120°C to about 260°C. However, for practical purposes commercial operations will normally be carried out in a temperature range of from about 150°C to about 200°C. Temperatures in the range of from about 160°C to about 190°C are most preferred.

Reactions can be carried out in the presence of any alkoxylating agent which produces a mole adduct of the sort desired. Normally such agents are alpha or beta alkylen

oxides. In most commercial operations either ethylene oxide, propylene oxide or mixtures of these will be used to produce an adduct. Of these products, ethylene oxide is most preferred.

Reaction products can have any desired content of alkoxylating agents such as ethylene oxide, but will normally range from about 30 to about 80% content of ethoxylating agent based on weight. For most purposes, the content of alkoxylating agent will range from about 40% to about 70% by weight. The amount of such materials present in the reaction is not critical other than the minimum amount necessary to provide sufficient units to reach the mole adduct level desired for the material being reacted.

The strontium-containing and barium-containing catalysts of the present invention are basic catalysts which provide the benefits of such catalysts while greatly reducing the amount of unreacted materials and undesirable by-products normally found in sharp distribution reactions. In addition, the instant invention provides that effective amounts of promoters can be added to the strontium-containing and barium-containing catalysts. These promoters are selected from the group consisting of phenols, acids, amines, amides, aldehydes, polyols, ketones, or alcohols in order to further reduce by-product reactions and to reduce or eliminate any induction periods necessary for alkoxylation to begin.

Representative examples of strontium-containing materials are strontium metal, strontium hydride, strontium oxide, strontium hydroxide, and strontium hydroxide.$XH_2O$ where X represents the number of water molecules present.

Representative examples of barium-containing materials are barium metal, barium hydride, barium oxide, barium hydroxide, and barium hydroxide.$XH_2O$ where X represents the number of water molecules present.

Many of the strontium and barium compounds alone are active in the process of the present invention and are extremely active when used with an effective amount of a co-catalyst or a promoter. Mixtures of these catalysts can be used.

When used, these catalyst mixtures can be used in any desired effective quantity. However, the larger the quantity used, the more quickly the reaction goes to completic Larger quantities do not appear to significantly alter the distribution obtained. For practical purposes, normally at least about 0.1% strontium or barium catalyst, based on the weight of the material to be reacted is present in the reaction, but up to about 5 weight percent is commonly used. Most commercial reactions will use from about 0.1 to about 2.0 weight percent based on the weight of the material to be reacted.

The amount of promoter or co-catalyst which should be present with a strontium or barium-containing catalyst is generally an effective amount. The effect of the co-catalyst or promoter becomes significant at somewhat above 0.1% by weight based on the weight of the material to be reacted. It is logical to expect an upper limit after which any promoter present produces no additional benefits. Normally, these materials are added to the strontium or barium catalysts in amounts ranging from about 0.1 to about 2% by weight based upon the weight of the material to be reacted. It is very apparent that these limits can be varied substantially.

While the instant invention is effective with all classes of polyols, aldehydes, ketones, amines, amides, acids, and mercaptans, it is preferred that the instant invention alkoxylate materials be selected from the group consisting of

a) polyols having boiling points above 100°C, a total of 2 to 30 carbon atoms and having 2 or more adjacent or non-adjacent hydroxyl containing moieties of the general formula

$$R_1 - \overset{\displaystyle R_2}{\underset{\displaystyle R_3}{\overset{|}{\underset{|}{C}}}} - OH$$

wherein $R_1$, $R_2$, and $R_3$ are, independently, linear or branched acyclic groups, alicyclic groups, aryl

groups, cyclic groups, or hydrogen, and wherein the R-designated groups can in addition contain one or more functional groups selected from the group consisting of ether, amine, carboxyl, halogen, nitro, carbonyl, and amide;

b) aldehydes and ketones having boiling points above 100°C, a total of from 2 to 30 carbon atoms, and having one or more adjacent or non-adjacent carbonyl containing moieties of the general formula

$$R_1 - \overset{O}{\overset{\|}{C}} - R_2$$

wherein $R_1$ and $R_2$ are, independently, hydrogen, linear or branched acyclic groups, alicyclic groups, cyclic groups, or aryl groups, and wherein the R-designated groups can in addition contain one or more functionalities selected from the group consisting of carboxyl, hydroxyl, ether, halogen, nitro, amine, or amide;

c) primary, secondary, or tertiary amides having boiling points above 100°C and a total of from 1 to 30 carbon atoms and containing 1 or more amide containing moeities of the general formula

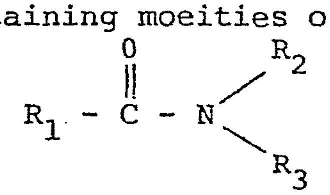

wherein $R_1$, $R_2$, and $R_3$ are, independently, hydrogen, linear or branched acyclic groups, alicyclic groups, cyclic groups, or aryl groups, and wherein the R-designated groups can in addition contain one or more other functionalities selected from the group consisting of hydroxyl, ether, carboxyl, carbonyl, amine, nitro, or halogen;

d) primary, secondary, or tertiary amines having a boiling point above 100°C, a total of from 1 to 30 carbon atoms and having 1 or more amine containing moieties of the general formula

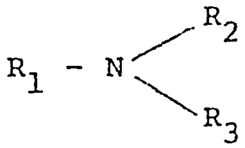

wherein $R_1$, $R_2$, and $R_3$ are, independently, hydrogen, linear or branched acyclic groups, alicyclic groups, cyclic groups, or aryl groups, and wherein the R-designated groups can in addition contain one or more functionalities selected from the group consisting of hydroxyl, ether, carbonyl, halogen, carboxyl, nitro, or amide; and

e) organic acids having boiling points above 100°C, a total of 1 to 30 carbon atoms and having 1 or more adjacent or non-adjacent carboxylic acid containing moieties of the general formula

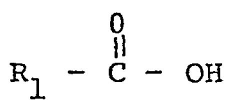

wherein $R_1$ is hydrogen, a linear or branched acyclic group, alicyclic group, cyclic group, or aryl group, and wherein the R group can in addition contain one or more functionalities selected from the group consisting of carbonyl, hydroxyl, halogen, ether, nitro, amine, or amide.

f) mercaptans of the general formula

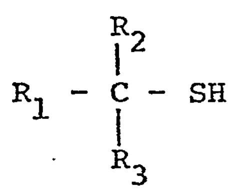

wherein $R_1$, $R_2$, and $R_3$ are, independently, hydrogen, linear or branched acyclic groups, alicyclic groups, aryl groups, or cyclic groups, and wherein the R-designated groups can in addition contain functional groups selected from the group consisting of halogen nitro, carboxyl, amine, carbonyl, ether, and amide.

It has also been discovered that the present inventi can be carried out even more efficiently and rapidly in the presence of a catalyst promoter. The catalyst promoter can be materials which are added to an alkoxylation system together w the catalyst or catalysts of choice. Representative examples

catalyst promoters useful in the process of the present invention are polyols, aldehydes, ketones, amides, amines, acids, phenols, mercaptans, and alcohols, wherein the alkoxylation is carried out at a temperature of from about 90°C to about 260°C.

Useful promoters for the strontium-containing and barium-containing catalysts of the present invention are

a) polyols having boiling points above 100°C, a total of 2 to 30 carbon atoms and having 2 or more adjacent or non-adjacent hydroxyl containing moieties of the general formula

$$R_1 - \underset{R_3}{\overset{R_2}{\underset{|}{\overset{|}{C}}}} - OH$$

wherein $R_1$, $R_2$, and $R_3$ are, independently, linear or branched acyclic groups, alicyclic groups, aryl groups, cyclic groups, or hydrogen, and wherein the R-designated groups can in addition contain one or more functional groups selected from the group consisting of ether, amine, carboxyl, halogen, nitro, carbonyl, and amide;

b) aldehydes and ketones having boiling points above 100°C, a total of from 2 to 30 carbon atoms and having one or more adjacent or non-adjacent carbonyl containing moieties of the general formula

$$R_1 - \overset{O}{\overset{\|}{C}} - R_2$$

wherein $R_1$, $R_2$ are, independently, hydrogen, linear or branched acyclic groups, alicyclic groups, cyclic groups, or aryl groups, and wherein the R-designated groups can in addition contain one or more functionalities selected from the group consisting of carboxyl, hydroxyl, ether, halogen, nitro, amine, or amide;

c) primary, secondary, or tertiary amides having boilin points above 100°C, a total of from 1 to 30 carbon atoms and containing 1 or more amide containing moieties of the general formula

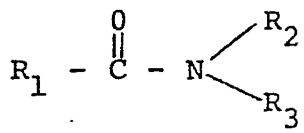

wherein $R_1$, $R_2$, and $R_3$ are, independently, hydrogen, linear or branched acyclic groups, alicyclic groups, cyclic groups, or aryl groups and wherein the R-designated groups can in addition contain one or more other functionalities selected from the group consisting of hydroxyl, ether, carboxyl, carbonyl, amine, nitro, or halogen;

d) primary, secondary, or tertiary amines having boiling points above 100°C, a total of 1 to 30 carbon atoms and containing 1 or more amine containi moieties of the general formula

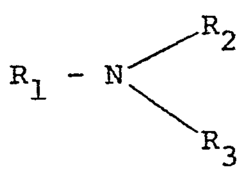

wherein $R_1$, $R_2$, and $R_3$, are, independently, hydroger linear or branched acyclic groups, alicyclic groups, cyclic groups, or aryl groups, and wherein the R-designated groups can in addition contain one or more functionalities selected from the group consisting of hydroxyl, ether, carbonyl, halogen, carboxyl, nitro, or amide; and

e) organic acids having boiling points above 100°C, a total of 1 to 30 carbon atoms and having 1 or more adjacent or non-adjacent carboxylic acid containing moieties of the general formula

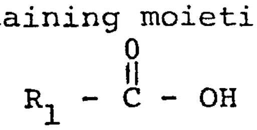

wherein $R_1$ is hydrogen, a linear or branched acyclic group, alicyclic group, cyclic group, or

aryl group, and wherein the R group can in addition contain one or more functionalities selected from the group consisting of carbonyl, hydroxyl, halogen, ether, nitro, amine, or amide;

f) phenols having boiling points of above 100°C, a total of from 6 to 30 carbon atoms and having 1 or more functionalities of the general formula

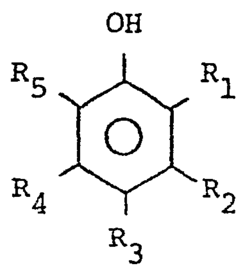

wherein $R_1$, $R_2$, $R_3$, $R_4$, and $R_5$ are, independently, hydrogen, halogen, hydroxyl, nitro, ether, or carbonyl, linear or branched acyclic groups, alicyclic groups, cyclic groups, aryl groups, or substituted aryl groups, and wherein in addition the R-designated groups can contain one or more functionalities selected from the group consisting of halogen, ether, nitro, carboxyl, carbonyl, amine, amide, or hydroxyl, (primary and secondary, linear and branched); and

g) mercaptans of the general formula

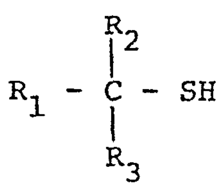

wherein $R_1$, $R_2$ and $R_3$ are, independently, hydrogen, linear or branched acyclic groups, alicyclic groups, cyclic groups, or aryl groups, and wherein the R-designated groups can in addition contain one or more functionalities selected from the group consisting of carboxyl, hydroxyl, ether, halogen, nitro, amine, or amide;

In some cases it is advantageous to preform or prereact the catalyst promoter with the catalyst prior to addition to the alkoxylation system. When this is carried

out, the desired amount of catalyst is reacted with an effective amount of promoter for a period of time ranging up to about 4 hours up to about 200°C. Normally however, such preforming or prereaction will be carried out at temperatures of from about 25°C to about 160°C for periods of time ranging from about 0.5 to about 2.5 hours. Such preformed catalysts can be isolated and recovered or in the preferred embodiment simply added as a reaction mixture to the major alkoxylation reaction. Many of the materials susceptible to alkoxylation reactions using the catalyst of the present invention are also effective promoters, both with each other and with other groups of materials.

Representative examples of various polyol reactants and/or promoters effective in the process of the present inve are

ethylene glycol
1,2-propylene glycol
1,4-butanediol
1,6-hexanediol
1,10-decanediol
1,3-butylene glycol
diethylene glycol
diethylene glycol monobutyl ether
diethylene glycol monomethyl ether
diethylene glycol monoethyl ether
dipropylene glycol
dipropylene glycol monomethyl ether
ethylene glycol monomethyl ether
ethylene glycol monoethyl ether
ethylene glycol monobutyl ether
hexylene glycol
pentaerythritol
dipentaerythritol
tripentaerythritol
trimethylolpropane
trimethylolethane
neopentyl glycol
diethanolamine
triethanolamine

diisopropanolamine

triisopropanolamine

1,4-dimethylolcyclohexane

2,2-bis(hydroxymethyl)propionic acid

1,2-bis(hydroxymethyl)benzene

4,5-bis(hydroxymethyl)furfural

4,8-bis(hydroxymethyl)tricyclo[5,2,1,0] decane

tartaric acid

2-ethyl-1,3-hexanediol

2-amino-2-ethyl-1,3-propanediol

triethylene glycol

tetraethylene glycol

glycerol

ascorbic acid, and

sugar alcohols selected from the group consisting of erythritol, dulcitol, sorbitol, mannitol and lanolin

Representative examples of various aldehydes and ketone reactants and/or promoters effective in the process of the instant invention are

lauryl aldehyde

benzaldehyde

2-undecanone

acetophenone

2,4-pentanedione

acetylsalicylic acid

ortho-chlorobenzaldehyde

para-chlorobenzaldehyde

cinnamic aldehyde

diisobutyl ketone

ethyl acetoacetate

ethylamyl ketone

camphor

para-hydroxybenzaldehyde

2-carboxybenzaldehyde

4-carboxybenzaldehyde

salicylaldehyde

octyl aldehyde

decyl aldehyde
p-methoxybenzaldehyde
p-aminobenzaldehyde
phenylacetaldehyde
acetoacetic acid
2,5-dimethoxybenzaldehyde
1-naphthyl aldehyde
terephthaldehyde

Representative examples of various amides useful in the process of the instant invention are

formamide
benzamide
acetanilide
salicylamide
acetoacetanilide
ortho-acetoacetotoluidide
acrylamide
N,N-diethyltoluamide
N,N-dimethylacetamide
N,N,-dimethylformamide
phthalimide
octylamide
decylamide
laurylamide
stearylamide
N,N-dimethylollaurylamide
N,N-dimethylacrylamide
para-chlorobenzamide
para-methoxybenzamide
para-aminobenzamide
para-hydroxybenzamide
ortho-nitrobenzamide
N-acetyl-para-aminophenol
2-chloroacetamide
oxamide
N,N-methylene-bis-acrylamide
dodecylamide

tetradecylamide
hexadecylamide
octadecylamide
octadecenylamide

Representative examples of various amines useful in the process of the instant invention are

aniline
benzylamine
hexadecylamine
triphenylamine
aminoacetic acid
anthranilic acid
cyclohexylamine
tert-octylamine
ortho-phenylenediamine
meta-phenylenediamine
para-phenylenediamine
N-acetyl-para-aminophenol
2-amino-4-chlorophenol
2-amino-2-ethyl-1,3-propanediol
ortho-aminophenol
para-aminophenol
para-aminosalicylic acid
benzyl-N,N-dimethylamine
tert-butylamine
2-chloro-4-aminotoluene
6-chloro-2-aminotoluene
meta-chloroaniline
ortho-chloroaniline
para-chloroaniline
4-chloro-2-nitroaniline
dibutylamine
2,5-dichloroaniline
3,4-dichloroaniline
dicyclohexylamine
diethanolamine
N,N-diethylethanolamine
N,N-diethyl-meta-toluidine

N,N-diethylaniline
diethylenetriamine
diisopropanolamine
N,N-dimethylethanolamine
N,N-dimethylaniline
2,4-dinitroaniline
diphenylamine
ethyl para-aminobenzoate
N-ethylethanolamine
N-ethyl-1-naphthylamine
N-ethyl-ortho-toluidine
N-ethylaniline
ethylenediamine
hexamethylenetetraamine
2,4-lutidine
N-methylaniline
methyl anthranilate
p,p'diaminodiphenylmethane
ortho-nitroaniline
para-nitroaniline
tert-octylamine
piperazine
ethanolamine
isopropanolamine
ortho-toluidine
para-toluidine
2,4-toluylenediamine
triethanolamine
tributylamine
triisopropanolamine
2,4-dimethylxylidine
para-methoxyaniline
nitrilotriacetic acid
N-phenyl-1-naphthylamine
decylamine
dodecylamine
tetradecylamine

hexadecylamine
octadecylamine
octadecenylamine
dilaurylamine
N-ethyl laurylamine

Representative examples of various acids useful in the process of the instant invention are

formic acid
acetic acid
valeric acid
heptanoic acid
2-ethylhexanoic acid
lauric acid
stearic acid
oleic acid
tall oil acids
hydrogenated tall oil acids
benzoic acid
salicylic acid
adipic acid
azelaic acid
fumaric acid
citric acid
acrylic acid
aminoacetic acid
para-aminosalicylic acid
anthranilic acid
butyric acid
propionic acid
ricinoleic acid
chloroacetic acid
ortho-chlorobenzoic acid
2,4-dichlorophenoxyacetic acid
tert-decanoic acid
para-aminobenzoic acid
abietic acid
itaconic acid
lactic acid

glycolic acid
malic acid
maleic acid
cinnamic acid
para-hydroxybenzoic acid
methacrylic acid
oxalic acid
myristic acid
palmitic acid
tert-pentanoic acid
phenylacetic acid
mandelic acid
sebacic acid
tallow fatty acids
hydrogenated tallow fatty acids
tartaric acid
trichloroacetic acid
2,4,5-trichlorophenoxyacetic acid
undecylenic acid
crotonic acid
pelargonic acid
acetoacetic acid
para-nitrobenzoic acid
ascorbic acid
nitrilotriacetic acid
naphthenic acids
1-naphthoic acid
trimellitic acid

Representative examples of various mercaptans in tl process of the instant invention are

dodecyl mercaptan
t-dodecyl mercaptan
isodecyl mercaptan
octyl mercaptan
octadecyl mercaptan

0046947

Representative examples of various phenols which are promoters for the catalysts of the present invention are

phenol
ortho-cresol
meta-cresol
2,4-dimethylphenol
2,5-dimethylphenol
2,6-dimethylphenol
para-cresol
ortho-chlorophenol
meta-chlorophenol
para-chlorophenol
para-nitrophenol
para-methoxyphenol
salicylic acid
meta-hydroxyacetophenone
para-aminophenol
ortho-phenylphenol
nonylphenol
octylphenol
t-butyl-para-cresol
hydroquinone
catechol
resorcinol
pyrogallol
1-naphthol
2-naphthol
4,4'-isopropylidenediphenol (bisphenol A)
methyl salicylate
benzyl salicylate
4-chloro-2-nitrophenol
para-t-butylphenol
2,4-di-t-amylphenol
2,4-dinitrophenol
para-hydroxybenzoic acid
8-hydroxyquinoline
methyl para-hydroxybenzoate
2-nitro-para-cresol

ortho-nitrophenol
para-phenylphenol
phenyl salicylate
salicylaldehyde
p-hydroxy benzaldehyde
2-amino-4-chlorophenol
ortho-aminophenol
salicylamide
2,4-dichlorophenol
2,5-dichlorophenol
2,5-dichlorohydroquinone

Certain alcohols can also act as promoters for ethoxylation of organic material using strontium-containing or barium-containing catalysts. Such alcohols are more acidic than normal alcohols such that the association consta is greater (or the pKa is lower) than normal alcohols. Thus, these alcohol promoters would have a pKa less than 17.

These alcohols are those containing a hydroxyl group and having the formula

$$R_1 - \overset{\displaystyle R_2}{\underset{\displaystyle R_3}{\overset{|}{\underset{|}{C}}}} - OH$$

wherein $R_1$, $R_2$, and $R_3$ are, independently, hydrogen, linear or branched acyclic groups, alicyclic groups, aryl groups, or cyclic groups, and wherein the R-designated groups can i addition contain functional groups selected from the group consisting of halogen, nitro, carboxyl, amine, carbonyl, ether, and amide. The molecule can contain a total of from 2 to 30 carbon atoms.

Representative examples of these effective alcoho promoters are:

triphenylmethanol
trichloroethanol
trifluoroethanol
2-nitroethanol

2-chloroethanol
2,2-dichloroethanol
2-methoxyethanol
2-chlorocyclohexanol
ortho-chlorobenzyl alcohol

The invention is more concretely described with reference to the examples below wherein all parts and percentages are by weight unless otherwise specified. The examples are provided to illustrate the instant invention and not to limit it.

Example 1

A 600 cubic centimeter (cc) stainless steel reactor was charged with 120 grams of heptanoic acid and 3.2 grams $Sr(OH)_2.8H_2O$ catalyst. After purging with nitrogen at 50 cc per minute for one hour at 150°C, the reactor was evacuated and the temperature raised to about 175°C. Ethylene oxide (EO) was then introduced to a total pressure of about 40 psig and EO uptake of 92.6 grams was allowed to proceed at this pressure. Ethoxylation was completed in 300 minutes after which the catalyst was neutralized.

Example 2

Example 1 was repeated except that 2 grams of barium hydroxide.$H_2O$ was used as catalyst. Ethylene oxide uptake of 180 grams was completed in 93 minutes. The catalyst was neutralized and the resulting ethoxylate product had a pour point of -60°F.

Example 3

An experiment was carried out as described in example 2 except that 1 gram barium hydroxide.$H_2O$ catalyst and 0.65 grams benzamide co-catalyst were used. Dehydration was accomplished by one hour of nitrogen purging at a rate of 50 to 100 cc per minute as the temperature increased from 100°C to 130°C. The reactor was then evacuated to 100 millimeters (mm) mercury at 130°C. EO uptake was completed in 123 minutes after which the catalyst was neutralized. The product had a pour point of -60°F.

Example 4

An experiment was carried out as described in example 1 except that 120 grams stearic acid (Hydrex 450, trademark of and sold by HUMKO Products, a division of KRAFTCO Corp) was used instead of heptanoic acid. After purging with nitrogen at 250cc per minute for one hour at 150°C, the reactor was evacuated and the temperature raised to about 178°C. EO addition of 61.3 grams was completed in 300 minutes after which the catalyst was neutralized.

Example 5

An experiment was carried out as described in Example 4 except that the catalyst was 3.2 grams of a preformed mixture of strontium hydroxide.$8H_2O$ (10.0 grams), phenol (7.8 grams), and water (17.8 grams). EO addition of 44 grams was completed in 315 minutes after which time the catalyst was neutralized.

Example 6

An experiment was carried out as described in example 2 except that 120 grams 2-ethylhexanoic acid was ethoxylated. One gram of barium hydroxide.$H_2O$ and benzamide (1.13 grams) were used as co-catalysts. The nitrogen purge rate was 100 cc per minute for dehydration. EO addition at 180 grams was completed in 73 minutes. The catalyst was neutralized to yield a product having a pour point of -60°F.

Example 7

An experiment was carried out as described in example 6 except that the co-catalyst was strontium hydroxide.8 (3.2 grams) and 1,2-propanediol (1.82 grams). EO addition of 110.9 grams was completed in 305 minutes.

Example 8

An experiment is carried out as described in example 7 except that co-catalysts are strontium hydroxide.$8H_2C$ (3.2 grams) and heptanoic acid (3.1 grams). EO addition is accomplished in about 300 minutes.

Example 9

A 600 cubic centimeter stainless steel reactor was charged with 150 grams propylene glycol, 0.5 grams barium

hydroxide $.H_2O$ and 0.42 grams nonanoic acid. After purging with nitrogen at 250cc per minute for 1 hour at 150°C, the reactor was evacuated and the temperature raised to about 178°C. 150 grams of ethylene oxide was added over 42 minutes at a reactor pressure of about 40 psig. The catalyst was neutralized to yield a product having a pour point of -60°F.

Example 10

An experiment was carried out as described in example 9 except that co-catalysts used were 2.0 grams barium hydroxide.$H_2O$ and 2.0 grams phenol. Ethylene oxide addition was completed in 25 minutes. Pour point of the product was -50°F after neutralization.

Example 11

An experiment was carried out as described in example 9 except that the reactor was charged with 60 grams of glycerol, 0.5 grams of barium hydroxide.$H_2O$, and 0.5 grams phenol. After purging, evacuation and heating to 178°C, propylene oxide (PO) was added to the reactor to a pressure of about 40 psig. Addition of 240 grams of PO was completed in 145 minutes. Pour point of the neutralized product was -15°F. Viscosities were 96.7 centistokes at 40°C and 8.17 centistokes at 100°C.

Example 12

An experiment was carried out as described in example 11 except that the reactor was charged with 10.0 grams glycerol, 1.0 grams barium hydroxide.$H_2O$, and 1.0 gram phenol. PO addition of 290 grams was completed in 207 minutes. The product had a pour point of -40°F and viscosities of 146.7 and 23.2 centistokes at 40°C and 100°C respectively.

Example 13

An experiment was carried out as described in example 9 except that the reactor was charged with 60 grams glycerol, 2.0 grams strontium hydroxide.$8H_2O$, and 1.82 grams benzamide. Addition of 240 grams EO was completed in 43 minutes. Pour point of the product was -35°F.

Example 14

An experiment was carried out as described in example 9 except the reactor was charged with 60 grams glycerol, 0.5 grams barium hydroxide.$H_2O$, and 0.57 grams benzylamine. Addition of 240 grams EO was completed in 71 minutes. Pour point of the product was -75°F.

Example 15

An experiment was carried out as described in example 9 except the reactor was charged with 60.0 grams sorbitol, 2.0 grams strontium hydroxide.$8H_2O$, and 1.28 grams 2-undecanone. Addition of 240 grams of EO was completed in 127 minutes. The product pour point was -15°F.

Example 16

An experiment was carried out as described in example 9 except the reactor was charged with sorbitol (60.0 grams), barium hydroxide.$H_2O$ (1.0 grams), and benzaldehyde (1.12 grams). At a reaction temperature of 178°C, ethylene oxide addition was completed to the extent desired at a reactor pressure of about 40 psig.

Example 17

An experiment was carried out as described in example 9 except the reactor was charged with 150 grams propylene glycol, barium hydroxide.$H_2O$ (0.5 grams), and glycerol (0.24 grams).

Example 18

A stainless steel reactor having 600 cc capacity was charged with 120 grams benzylamine, 2.0 grams barium hydroxide .$H_2O$, and 2.0 grams phenol. The reaction mass was purged with nitrogen at 50 cc per minute for one hour at 150°C. The reactor was then evacuated and temperature raised to about 178°C. 180 grams of ethylene oxide was added over 49 minutes at a reactor pressure between 20 and 40 psig. The catalyst was neutralized and the product had a pour point of -10°F.

Example 19

An experiment was carried out as described in example 18 except the reactor was charged with 150 grams benzylamine, 2.0 grams strontium hydroxide.$8H_2O$, and 2.2 grams 2-ethylhexanoic acid.

Example 20

An experiment is carried out as described in example 18 except the reactor is charged with 150 grams benzylamine, 2.0 grams barium hydroxide.$H_2O$ and 1.6 grams propylene glycol.

Example 21

An experiment was carried out as described in example 9 except the reactor was charged with 120 grams benzamide, 1.6 grams strontium hydroxide.$8H_2O$, and 1.2 grams phenol. Addition of 113.5 grams EO was completed in 210 minutes.

Example 22

An experiment is carried out as described in example 21 except the reactor is charged with 120 grams benzamide, 2.0 grams barium hydroxide.$H_2O$, and 3.0 grams 2-ethyl hexanoic acid.

Example 23

An experiment is carried out as described in example 21 except the reactor is charged with 120 grams benzamide, 2.0 grams barium hydroxide.$H_2O$, and 1.6 grams 1,2-propanediol.

Example 24

A 600 cc stainless steel reactor was charged with 120 grams of 1-dodecanethiol, 0.6 grams barium hydroxide.$H_2O$. After purging with nitrogen at 250cc per minute for 1 hour at 150°C, the reactor was evacuated and the temperature raised to about 170°C. EO was then introduced to a total pressure of about 40 psig and EO uptake of 180 grams was allowed to proceed at this pressure. Ethoxylation was completed in 55 minutes after which the catalyst was neutralize The product had a pour point of 50°F and contained 2.3% unreacted 1-dodecanethiol.

Example 25

An experiment was carried out as described in example 24 except the reactor was charged with 78 grams of 1-dodecanethiol and 0.6 grams barium hydroxide.$H_2O$. Addition of 117 grams of ethylene oxide was completed in 20 minutes. The product had a pour point of 60°F.

Example 26

An experiment was carried out as described in example 18 except the reactor was charged with 89 grams of lauryl aldehyde, 3.2 grams of strontium hydroxide.$8H_2O$, and 3.1 grams heptanoic acid. EO addition of 133.5 grams was completed in 150 minutes.

Example 27

An experiment is carried out as described in example 26 except the reactor is charged with 100 grams lauryl aldehyde, 3.0 grams barium hydroxide.$H_2O$, and 3.0 grams phenol.

Example 28

An experiment is carried out as described in example 26 except the reactor is charged with 100 grams lauryl aldehyde, 3.0 grams barium hydroxide.$H_2O$, and 2.6 grams propylene glycol.

Example 29

As a comparative example, a stainless steel reactor having 600 cc capacity was charged with 120 grams of alcohol (ALFOL 12 alcohol, a 12 carbon alcohol trademark of and sold by Conoco Inc.) and 0.15 grams sodium hydroxide. After purging with nitrogen at 250cc per minute for 1 hour at 150°C, the reactor was evacuated and the temperature was raised to about 178°C. Propylene oxide was then introduced to a total pressure of about 40 psig and propylene oxide uptake of 94 grams was allowed to proceed at this pressure. PO addition was completed in 124 minutes. After propoxylation, the catalyst was neutralized. Pour point of the product was 0°F.

Thus the instant invention provides a method for obtaining high mole adduct alkoxylates of polyols, aldehydes,

BAD ORIGINAL

ketones, amides, amines, acids, and mercaptans by contacting such materials with barium-containing and strontium-containing catalysts. Products obtained contain low amounts of by-products and unreacted organic materials.

Although exemplified as batch reactions, the catalyst and promoters of the present invention are extremely well suited to continuous reaction methods. The reaction products are of extremely high quality and quantity.

While certain embodiments and details have been shown for the purpose of illustrating this invention, it will be apparent to those skilled in this art that various changes and modifications may be made herein without departing from the spirit or scope of the invention.

We claim:

1. A method for the alkoxylation of a material selected from the group consisting of polyols, aldehydes, ketones, amides, amines, acids, and mercaptans, comprising contacting said material with an alkoxylating agent in the presence of at least one catalyst selected from the group consisting of strontium metal, barium metal, strontium oxide, barium oxide, strontium hydroxide, hydrated strontium hydroxide, barium hydroxide, hydrated barium hydroxide, strontium hydride, barium hydride, or mixtures of these wherein the alkoxylation is carried out at a temperature of from about 90°C to about 260°C.

2. A method as described in claim 1 wherein the alkoxylating agent is selected from the group consisting of ethylene oxide, propylene oxide, or mixtures of these.

3. A method as described in claim 2 wherein the alkylene oxide adduct ratio ranges from about 10 weight percent to about 80 weight percent based upon the total weight of the ethoxylated product.

4. A method as described in claim 3 wherein the reaction is carried out at pressures up to about 100 pounds per square inch gauge.

5. A method as described in claim 4 wherein the alkoxylating agent is ethylene oxide.

6. A method as described in claim 5 wherein the material ethoxylated is selected from the group consisting of

a) polyols having a boiling point above 100°C, a total of 2 to 30 carbon atoms and having 2 or more adjacent or non-adjacent hydroxyl-containing moieties of the general formula

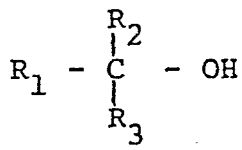

wherein $R_1$, $R_2$, and $R_3$ are, independently, linear or branched acyclic groups, alicyclic groups, aryl groups, cyclic groups, or hydrogen and wherein the R-designated groups can in addition contain one or more functional groups selected from the group consisting of ether, amine, carboxyl, halogen, nitro, carbonyl, and amide;

b) aldehydes and ketones having boiling points above 100°C, a total of from 2 to 30 carbon atoms, and having one or more adjacent or non-adjacent carbonyl containing moieties of the general formula

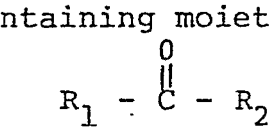

wherein $R_1$ and $R_2$ are, independently, hydrogen linear or branched acyclic groups, alicyclic groups, cyclic groups, or aryl groups and wherein the R-designated groups can in addition contain one or more functionalities selected from the group consisting of carboxyl, hydroxyl, ether, halogen, nitro, amine, or amide;

c) primary, secondary, or tertiary amides having boiling points above 100°C and a total of from 1 to 30 carbon atoms and containing one or more amide-containing moieties of the general formula

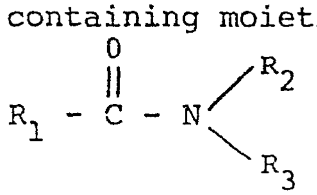

wherein $R_1$, $R_2$, and $R_3$ are, independently, hydrogen, linear or branched acyclic groups, alicyclic groups,

cyclic groups, or aryl groups, and wherein the R-designated groups can in addition contain one or more other functionalities selected from the group consisting of hydroxyl, ether, carboxyl, carbonyl, amine, nitro, or halogen;

d) primary, secondary, or tertiary amines having boiling points above 100°C, a total of from 1 to 30 carbon atoms and having one or more amine-containing moieties of the general formula

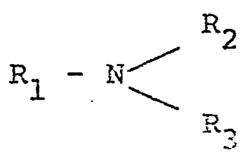

wherein $R_1$, $R_2$, and $R_3$ are, independently, hydrogen, linear or branched acyclic groups, alicyclic groups, cyclic groups, or aryl groups, and wherein the R-designated groups can in addition contain one or more functionalities selected from the group consisting of hydroxyl, ether, carbonyl, halogen, carboxyl, nitro, or amide; and

e) organic acids having boiling points above 100°C, a total of 1 to 30 carbon atoms and having one or more adjacent or non-adjacent carboxylic acid containing moieties of the general formula

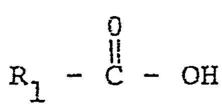

wherein $R_1$ is hydrogen, a linear or branched acyclic group, alicyclic group, cyclic group, or aryl group, and wherein the R group can in addition contain one or more functionalities selected from the group consisting of carbonyl, hydroxyl, halogen, ether, nitro, amine, or amide;

f) mercaptans of the general formula

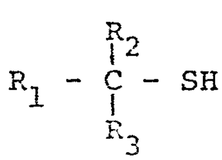

wherein $R_1$, $R_2$, and $R_3$ are, independently, hydrogen, linear or branched acyclic groups, alicyclic groups,

aryl groups, or cyclic groups, and wherein the R-designated groups can in addition contain functional groups selected from the group consisting of halogen, nitro, carboxyl, amine, carbonyl, ether, and amide.

7. A method for the alkoxylation of a material selected from the group consisting of polyols, aldehydes, ketones, amides, amines, acids, and mercaptans, comprising contacting said material with an alkoxylating agent in the presence of at least one catalyst selected from the group consisting of strontium metal, barium metal, strontium oxide, barium oxide, strontium hydroxide, hydrated strontium hydroxide, barium hydroxide, hydrated barium hydroxide, strontium hydride, barium hydride, or mixtures of these together with an effective amount of at least one catalyst promoting material selected from the group consisting of polyols, aldehydes, ketones, amides, amines, acids, phenols, mercaptans, and alcohols, wherein the alkoxylation is carried out at a temperature of from about 90°C to about 260°C.

8. A method as described in claim 7 wherein the alkoxylating agent is selected from the group consisting of ethylene oxide, propylene oxide, or mixtures of these.

9. A method as described in claim 8 wherein the alkylene oxide adduct ratio ranges from about 10 weight percent to about 80 weight percent based upon the total weight of the ethoxylated product.

10. A method as described in claim 9 wherein the reaction is carried out at pressures of up to about 100 psig.

11. A method as described in claim 10 wherein the alkoxylating agent is ethylene oxide.

12. A method as described in claim 11 wherein the barium-containing and/or strontium-containing catalysts are promoted by materials selected from the group consisting of

a) polyols having a boiling point above 100°C, a total of 2 to 30 carbon atoms and having 2 or more adjacent or non-adjacent hydroxyl-containing moieties of the general formula

$$R_1 - \overset{\displaystyle R_2}{\underset{\displaystyle R_3}{\overset{|}{\underset{|}{C}}}} - OH$$

wherein $R_1$, $R_2$, and $R_3$ are, independently, linear or branched acyclic groups, alicyclic groups, aryl groups, cyclic groups, or hydrogen, and wherein the R-designated groups can in addition contain one or more functional groups selected from the group consisting of ether, amine, carboxyl, halogen, nitro, carbonyl, and amide;

b) aldehydes and ketones having boiling points above 100°C, a total of from 2 to 30 carbon atom and having one or more adjacent or non-adjacent carbonyl-containing moieties of the general formula

$$R_1 - \overset{\displaystyle O}{\overset{\|}{C}} - R_2$$

wherein $R_1$ and $R_2$ are, independently, hydrogen, linear or branched acyclic groups, alicyclic groups, cyclic groups, or aryl groups, and wherein the R-designated groups can in addition contain one or more functionalities selected from the group consisting of carboxyl, hydroxyl, ether, halogen, nitro, amine, or amide;

c) primary, secondary, or tertiary amides having boiling points above 100°C, a total of from 1 to 30 carbon atoms and containing 1 or more amide-containing moieties of the general formula

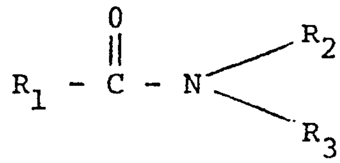

wherein $R_1$, $R_2$, and $R_3$ are, independently, hydrogen linear or branched acyclic groups, alicyclic groups cyclic groups, or aryl groups, and wherein the R-designated groups can in addition contain one or more other functionalities selected from the group consisting of hydroxyl, ether, carboxyl, carbonyl, amine, nitro, or halogen;

d) primary, secondary, or tertiary amines having boiling points above 100°C, a total of 1 to 30 carbon atoms and containing one or more amine-containing moieties of the general formula

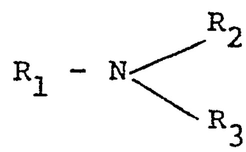

wherein $R_1$, $R_2$ and $R_3$ are, independently, hydrogen, linear or branched acyclic groups, alicyclic groups, cyclic groups, or aryl groups, and wherein the R-designated groups can in addition contain one or more functionalities selected from the group consisting of hydroxyl, ether, carbonyl, halogen, carboxyl, nitro, or amide; and

e) organic acids having boiling points above 100°C, a total of 1 to 30 carbon atoms and having one or more adjacent or non-adjacent carboxylic acid-containing moieties of the general formula

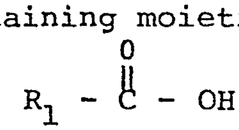

wherein $R_1$ is hydrogen, a linear or branched acyclic group, alicyclic group, cyclic group, or aryl group, and wherein the R group can in addition contain one or more functionalities selected from the group

consisting of carbonyl, hydroxyl, halogen, ether, nitro, amine, or amide;

f) mercaptans of the general formula

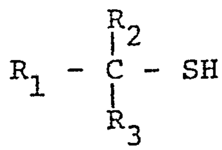

wherein $R_1$, $R_2$, and $R_3$ are, independently, hydrogen, linear or branched acyclic groups, alicyclic groups, aryl groups, or cyclic groups, and wherein the R-designated groups can in addition contain functional groups selected from the group consisting of halogen, nitro, carboxyl, amine, carbonyl, ether, and amide;

g) phenols having boiling points of above 100°C, a total of from 6 to 30 carbon atoms and having one or more functionalities of the general formula

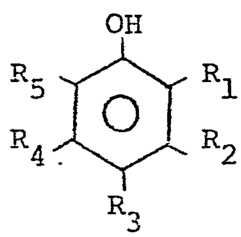

wherein $R_1$, $R_2$, $R_3$, $R_4$, and $R_5$ are, independently hydrogen, halogen, hydroxyl, nitro, ether, or carbonyl, linear or branched acyclic groups, alicyclic groups, cyclic groups, aryl groups, or substituted aryl groups, and wherein in addition the R-designated groups can contain one or more functionalities selected from the group consisting of halogen, ether, nitro, carboxyl, carbonyl, amine, amide, or hydroxyl; and

h) alcohols containing from 2 to 30 carbon atoms of the general formula

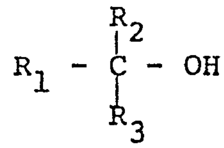

wherein $R_1$, $R_2$, and $R_3$ are, independently, hydrogen, linear or branched acyclic groups, alicyclic groups, aryl groups, or cyclic groups, and wherein the R-designated groups can in addition contain functional groups selected from the group consisting of halogen, nitro, carboxyl, amine, carbonyl, ether, and amide.

13. A method as described in claim 12 wherein catalysts are combined with promoters prior to carrying out said reactio said combining carried out at temperatures of up to 200°C for a time sufficient to effect said combination.

14. A method as described in claim 12 wherein the polyol is selected from the group consisting of

ethylene glycol
1,2-propylene glycol
1,4-butanediol
1,6-hexanediol
1,10-decanediol
1,3-butylene glycol
diethylene glycol
diethylene glycol monobutyl ether
diethylene glycol monomethyl ether
diethylene glycol monoethyl ether
dipropylene glycol
dipropylene glycol monomethyl ether
ethylene glycol monomethyl ether
ethylene glycol monoethyl ether
ethylene glycol monobutyl ether
hexylene glycol
pentaerythritol
dipentaerythritol

tripentaerythritol
trimethylolpropane
trimethylolethane
neopentyl glycol
diethanolamine
triethanolamine
diisopropanolamine
triisopropanolamine
1,4-dimethylolcyclohexane
2,2-bis(hydroxymethyl)propionic acid
1,2-bis(hydroxymethyl)benzene
4,5-bis(hydroxymethyl)furfural
4,8-bis(hydroxymethyl)tricyclo[5,2,1,0] decane
tartaric acid
2-ethyl-1,3-hexanediol
2-amino-2-ethyl-1,3-propanediol
triethylene glycol
tetraethylene glycol
glycerol
ascorbic acid, and
sugar alcohols selected from the group consisting of erythritol, dulcitol, sorbitol, mannitol and lanolin

15. A method as described in claim 12 wherein the aldehydes and ketones are selected from the group consisting of

lauryl aldehyde
benzaldehyde
2-undecanone
acetophenone
2,4-pentanedione
acetylsalicylic acid
ortho-chlorobenzaldehyde
para-chlorobenzaldehyde
cinnamic aldehyde
diisobutyl ketone
ethyl acetoacetate
ethylamyl ketone

camphor
para-hydroxybenzaldehyde
2-carboxybenzaldehyde
4-carboxybenzaldehyde
salicylaldehyde
octyl aldehyde
decyl aldehyde
p-methoxybenzaldehyde
p-aminobenzaldehyde
phenylacetaldehyde
acetoacetic acid
2,5-dimethoxybenzaldehyde
l-naphthyl aldehyde
terephthaldehyde

16. A method as described in claim 12 wherein the amides are selected from the group consisting of
formamide
benzamide
acetanilide
salicylamide
acetoacetanilide
ortho-acetoacetotoluidide
acrylamide
N,N-diethyltoluamide
N,N-dimethylacetamide
N,N,-dimethylformamide
phthalimide
octylamide
decylamide
laurylamide
stearylamide
N,N-dimethylollaurylamide
N,N-dimethylacrylamide
para-chlorobenzamide
para-methoxybenzamide
para-aminobenzamide

para-hydroxybenzamide
ortho-nitrobenzamide
N-acetyl-para-aminophenol
2-chloroacetamide
oxamide
N,N-methylene-bis-acrylamide
dodecylamide
tetradecylamide
hexadecylamide
octadecylamide
octadecenylamide

17. A method as described in claim 12 wherein the amines are selected from the group consisting of
aniline
benzylamine
hexadecylamine
triphenylamine
aminoacetic acid
anthranilic acid
cyclohexylamine
tert-octylamine
ortho-phenylenediamine
meta-phenylenediamine
para-phenylenediamine
N-acetyl-para-aminophenol
2-amino-4-chlorophenol
2-amino-2-ethyl-1,3-propanediol
ortho-aminophenol
para-aminophenol
para-aminosalicylic acid
benzyl-N,N-dimethylamine
tert-butylamine
2-chloro-4-aminotoluene
6-chloro-2-aminotoluene
meta-chloroaniline
ortho-chloroaniline

para-chloroaniline
4-chloro-2-nitroaniline
dibutylamine
2,5-dichloroaniline
3,4-dichloroaniline
dicyclohexylamine
diethanolamine
N,N-diethylethanolamine
N,N-diethyl-meta-toluidine
N,N-diethylaniline
diethylenetriamine
diisopropanolamine
N,N-dimethylethanolamine
N,N-dimethylaniline
2,4-dinitroaniline
diphenylamine
ethyl para-aminobenzoate
N-ethyl ethanolamine
N-ethyl-1-naphthylamine
N-ethyl-ortho-toluidine
N-ethylaniline
ethylenediamine
hexamethylenetetraamine
2,4-lutidine
N-methylaniline
methyl anthranilate
p,p'diaminodiphenylmethane
ortho-nitroaniline
para-nitroaniline
tert-octylamine
piperazine
ethanolamine
isopropanolamine
ortho-toluidine
para-toluidine
2,4-toluylenediamine

triethanolamine
tributylamine
triisopropanolamine
2,4-dimethylxylidine
para-methoxyaniline
nitrilotriacetic acid
N-phenyl-1-naphthylamine
decylamine
dodecylamine
tetradecylamine
hexadecylamine
octadecylamine
octadecenylamine
dilaurylamine
N-ethyllaurylamine

18. A method as described in claim 12 wherein the acids are selected from the group consisting of
formic acid
acetic acid
valeric acid
heptanoic acid
2-ethylhexanoic acid
lauric acid
stearic acid
oleic acid
tall oil acids
hydrogenated tall oil acids
benzoic acid
salicylic acid
adipic acid
azelaic acid
fumaric acid
citric acid
acrylic acid
aminoacetic acid
para-aminosalicylic acid
anthranilic acid

butyric acid
propionic acid
ricinoleic acid
chloroacetic acid
ortho-chlorobenzoic acid
2,4-dichlorophenoxyacetic acid
tert-decanoic acid
para-aminobenzoic acid
abietic acid
itaconic acid
lactic acid
glycolic acid
malic acid
maleic acid
cinnamic acid
para-hydroxybenzoic acid
methacrylic acid
oxalic acid
myristic acid
palmitic acid
tert-pentanoic acid
phenylacetic acid
mandelic acid
sebacic acid
tallow fatty acids
hydrogenated tallow fatty acids
tartaric acid
trichloroacetic acid
2,4,5-trichlorophenoxyacetic acid
undecylenic acid
crotonic acid
pelargonic acid
acetoacetic acid
para-nitrobenzoic acid
ascorbic acid
nitrilotriacetic acid
naphthenic acids
1-naphthoic acid
trimellitic acid

19. A method as described in claim 12 wherein the mercaptans are selected from the group consisting of dodecyl mercaptan, t-dodecyl mercaptan, isodecyl mercaptan, octyl mercaptan, octadecyl mercaptan.

20. A method as described in claim 12 wherein the alcohols are selected from the group consisting of
triphenylmethanol
trichloroethanol
trifluoroethanol
2-nitroethanol
2-chloroethanol
2,2-dichloroethanol
2-methoxyethanol
2-chlorocyclohexanol
ortho-chlorobenzyl alcohol

0046947

European Patent Office

# EUROPEAN SEARCH REPORT

Application number

EP 81 10 6520

## DOCUMENTS CONSIDERED TO BE RELEVANT

| Category | Citation of document with indication, where appropriate, of relevant passages | Relevant to claim |
|---|---|---|
| | GB - A - 1 177 636 (I.C.I.)<br>* Whole document *<br>-- | 1,2,4-8,10-13,17 |
| PA | EP - A - 0 026 546 (UNION CARBIDE)<br>* Examples; claims *<br>-- | 1,2,4-8,10-13 |
| A | US - A - 3 761 523 (R.E. REID et al.)<br>* Whole document *<br>-- | 7,8,10-13 |
| A | US - A - 4 210 764 (K. YANG et al.)<br>* Claims *<br>-- | 1-13 |
| PA | US - A - 4 223 164 (K. YANG et al.)<br>* Claims *<br>-- | 1-13 |
| A | US - A - 3 839 212 (F.C. McCOY)<br>* Column 3, line 3 - column 4, line 19 *<br>---- | 1 |

CLASSIFICATION OF THE APPLICATION (Int. Cl.³)

C 07 B 29/00
C 08 G 65/28
65/26
B 01 J 23/02
C 07 C 41/03
43/13
45/64
85/24
102/00
148/00
51/367

TECHNICAL FIELDS SEARCHED (Int. Cl.³)

C 07 B 29/00
C 08 G 65/26
C 07 C 41/03
43/13
C 08 G 65/28
B 01 J 23/02

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 23-11-1981 | FLETCHER |